# EUROPEAN PATENT APPLICATION

(11) **EP 1 493 448 A1**
(43) Date of publication of application: **05.01.2005**
(21) Application number: 03745697.7
(22) Date of filing: 03.04.2003
(51) Int. Cl.: A61K 45/06, A61K 31/404, A61K 31/427, A61K 31/357, A61K 31/496, A61K 31/4439, A61K 31/421, A61K 31/4245, A61K 31/426, A61K 31/4709, A61K 31/538, A61P 9/00, A61P 9/10

(54) **MEDICINAL COMPOSITION COMPRISING ACAT INHIBITOR AND INSULINE RESISTANCE IMPROVING AGENT**

(30) Priority: 05.04.2002 JP 2002103134
(71) Applicant: Sankyo Company, Limited, Tokyo 103-8426 (JP)
(72) Inventor: INABA, Toshimori, SANKYO COMPANY LIMITED, Tokyo 140-8710 (JP); FUJIWARA, Toshihiko, SANKYO COMPANY LIMITED, Tokyo 140-8710 (JP)
(74) Representative: Gibson, Christian John Robert
(86) International application number: PCT/JP2003/004296
(87) International publication number: WO 2003/084572

(57) **Abstract**

The present invention provides a pharmaceutical composition comprising an ACAT inhibitor and an insulin resistance reducing agent as effective components for the prevention or therapy against atherosclerosis or diseases caused by atherosclerosis.

## Description

### [TECHNICAL FIELD]

The present invention relates to a pharmaceutical composition comprising an acyl-coenzyme A: cholesterol acyltransferase (hereinafter referred to as ACAT) inhibitor and an insulin resistance reducing agent for prevention or therapy of atherosclerosis and diseases caused by atherosclerosis such as ischemic heart diseases, ischemic brain diseases and peripheral circulatory disorders, that is characterized by said composition being formulated to provide administration of these two agents at the same time or independently at a particular time interval.

Furthermore, the present invention relates to a method of prevention or therapy of atherosclerosis and diseases caused by atherosclerosis such as ischemic heart diseases, ischemic brain diseases and peripheral circulatory disorders by administering to a warm-blooded animal (especially a human) a pharmaceutically effective dose of a pharmaceutical composition comprising an ACAT inhibitor and an insulin resistance reducing agent formulated to provide administration of these two agents at the same time or independently at a particular time interval.

### [BACKGROUND OF THE INVENTION]

In accordance with Westernization of diet and a progressive increase in the elderly population in Japan, the number of patients with atherosclerosis is increasing. Atherosclerosis is a main cause of ischemic heart diseases (myocardial infarct, unstable angina and ischemic sudden death), ischemic brain diseases (cerebral infarction and cerebral hemorrhage), peripheral circulatory disorders, and the like. In addition to hyperlipidemia (particularly hypercholesterolemia), hypertension and abnormal glucose metabolism centered on insulin-resistance have been pointed out as risk factors for developing atherosclerosis. These risk factors appear to induce complications (syndrome X) in many cases. For instance, hyperlipidemia is a risk factor not only for atherosclerosis but also for xanthomatosis. Particularly, in advanced cases of atherosclerosis, xanthomatosis and vascular complications such as myocardial infarction are considered likely to develop. These pathological causes are closely related with each other (Diabetes 37 1595 (1988)). Thus effective prophylactic and therapeutic agents are required.

It has been disclosed that insulin resistance reducing agents such as troglitazone, pioglitazone and the like are more beneficial in patients with Alzheimer's disease when an ACAT inhibitor is co-administered than when administered alone (WO99/38498). However, no inhibitory effects of co-administration of an ACAT inhibitor with an insulin resistance reducing agent on the progressive development of atherosclerosis and on the appearance of xanthomatosis in the arthrosis of four limbs have been observed.

### [DISCLOSURE OF THE INVENTION]

The present inventors have studied therapeutic agents against atherosclerosis and found that pharmaceutical compositions comprising an ACAT inhibitor and an insulin resistance reducing agent exhibit more potent inhibitory activities against the progressive development of atherosclerosis as well as the appearance of xanthomatosis in the arthrosis of four limbs when these agents are administered at the same time or separately at a particular time interval than obtained by administration of either agent alone. Since these pharmaceutical compositions have low toxicity, these pharmaceutical compositions are concluded to be useful as prophylactic or therapeutic agents (particularly as a therapeutic agent) in warm-blooded animals (particularly in humans) with atherosclerosis or diseases caused by atherosclerosis such as ischemic heart diseases ischemic brain diseases and peripheral circulatory disorders. Thus the present inventors have completed the present invention.

The present invention relates to
(1) a pharmaceutical composition comprising an ACAT inhibitor and an insulin resistance reducing agent for prevention or therapy against atherosclerosis or diseases caused by atherosclerosis, which is characterized by said composition being formulated to provide administration of these two agents at the same time or independently at a particular time interval.
   Of said pharmaceutical compositions, examples of preferred pharmaceutical compositions include:
(2) a pharmaceutical composition comprising an ACAT inhibitor such as FR-129169, CI-1011, F-1394, F-12511, T-2591, FCE-28654, K-10085, HL-004, NTE-122, FR-186054, N-(1-pentyl-4,6-dimethylindolin-7-yl)-2,2-dimethylpropanamide or N-(1-octyl-5-carboxymethyl-4,6-dimethylindolin-7-yl)-2,2-dimethylpropanamide, and pharmaceutically acceptable salts thereof,
(3) a pharmaceutical composition comprising an ACAT inhibitor such as CI-1011, F-12511, N-(1-pentyl-4,6-dimethylindolin-7-yl)-2,2-dimethylpropanamide or N-(1-octyl-5-carboxymethyl-4,6-dimethylindolin-7-yl)-2,2-dimethyl-propanamide, and pharmaceutically acceptable salts thereof,
(4) a pharmaceutical composition comprising an ACAT inhibitor such as N-( 1-octyl-5-carboxymethyl-4,6-dimethylindolin-7-yl)-2,2-dimethyl-propanamide and pharmaceutically acceptable salts thereof,
(5) a pharmaceutical composition comprising an insulin resistance reducing agent such as pioglitazone, rosiglitazone, GI-262570, JTT-501, AZ-242, MCC-555, YM-440, KRP-297, T-174, NC-2100, NN-622, BMS-298585 and 5-[4-(6-methoxy-1-methylbenzimidazol-2-ylmethoxy)benzyl]thiazolidine-2,4-dione, and pharmaceutically acceptable salts thereof,
(6) a pharmaceutical composition comprising an insulin resistance reducing agent such as pioglitazone, rosiglitazone or 5-[4-(6-methoxy-1-methylbenzimidazol-2-ylmethoxy)benzyl]-thiazolidine-2,4-dione, and pharmaceutically acceptable salts thereof, and
(7) a pharmaceutical composition comprising an insulin resistance reducing agent such as 5-[4-(6-methoxy-1-methylbenzimidazol-2-ylmethoxy)benzyl]-thiazolidine-2,4-dione and pharmaceutically acceptable salts thereof.
   Furthermore, pharmaceutical compositions comprising arbitrary combinations of an ACAT inhibitor selected from those listed in (2) to (4) and an insulin resistance reducing agent selected from (5) to (7) are preferred. The following pharmaceutical compositions, for example, are included in preferred compositions;
(8) a pharmaceutical composition wherein the ACAT inhibitor, which is an effective component thereof, is FR-129169, CI-1011, F-1394, F-12511, T-2591, FCE-28654, K-10085, HL-004, NTE-122, FR-186054, N-(1-pentyl-4,6-dimethylindolin-7-yl)-2,2-dimethylpropanamide, or N-(1-octyl-5-carboxymethyl-4,6-dimethylindolin-7-yl)-2,2-dimethylpropanamide, or a pharmaceutically acceptable salt thereof, while the insulin resistance reducing agent, which is another effective component thereof, is pioglitazone, rosiglitazone, GI-262570, JTT-501, AZ-242, MCC-555, YM-440, KRP-297, T-174, NC-2100, NN-622, BMS-298585, or 5-[4-(6-methoxy-1-methylbenzimidazol-2-ylmethoxy)benzyl]thiazolidine-2,4-dione, or a pharmaceutically acceptable salt thereof,
(9) a pharmaceutical composition wherein the ACAT inhibitor, which is an effective component thereof, is CI-1011, F-12511, N-(1-pentyl-4,6-dimethylindolin-7-yl)-2,2-dimethylpropanamide, or N-(1-octyl-5-carboxymethyl-4,6-dimethylindolin-7-yl)-2,2-dimethyl-propanamide, or a pharmaceutically acceptable salt thereof, while the insulin resistance reducing agent, which is another effective component thereof, is pioglitazone, rosiglitazone, or 5-[4-(6-methoxy-1-methylbenzimidazol-2-ylmethoxy)benzyl]thiazolidine-2,4-dione, or a pharmaceutically acceptable salt thereof, or
(10) a pharmaceutical composition wherein the ACAT inhibitor, which is an effective component thereof, is N-(1-octyl-5-carboxymethyl-4,6-dimethylindolin-7-yl)-2,2-dimethylpropanamide or a pharmaceutically acceptable salt thereof, while the insulin resistance reducing agent, which is another effective component thereof, is 5-[4-(6-methoxy-1-methylbenzimidazol-2-ylmethoxy)benzyl]-thiazolidine-2,4-dione or a pharmaceutically acceptable salts thereof.

The ACAT inhibitors, which are one of the effective components of the pharmaceutical composition of the present invention, are compounds which inhibit activity of acyl-coenzyme A: cholesterol acyltransferase (ACAT) and, as a result, decrease cholesterol level. Furthermore, since these compounds act directly at atherosclerotic lesions in the vascular wall and suppress foam cell formation from macrophages (i.e. the accumulation of cholesterol in cells), ACAT inhibitors are essentially used for the prevention or therapy of atherosclerosis. As such ACAT inhibitors, the following compounds, for example, are included:
Compounds having general formula (I) described in WO 92/09561 [FR-129169 is preferred and its chemical name is N-(1,2-diphenylethyl)-2-(2-octyloxyphenyl)-acetamide],
Compounds having the general formula (I) described in Japanese Patent Publication (Kohyo) Hei 8-510256 (WO 94/26702 or USP 5,491,172) and pharmaceutically acceptable salts thereof (CI-1011 is preferred and its chemical name is 2,6-diisopropylphenyl-N-[(2,4,6-triisopropylphenyl)acetyl]sulfamate and CI-1011 in the present invention includes its pharmaceutically acceptable salts),
Compounds having the general formula (I) described in EP 421 441 (USP 5,120,738) and pharmaceutically acceptable salts thereof (F-1394 is preferred and its chemical name is (1S,2S)-2-[3-(2,2-dimethylpropyl)-3-nonylureido]cyclohexan-1-yl-3-[(4R)-N-(2,2,5,5-tetramethyl-1,3-dioxane-4-carbonyl)amino]propionate and F-1394 in the present invention includes its pharmaceutically acceptable salts),
Compounds described in Japanese Patent Publication (Kohyo) Hei 2000-500771 (WO 97/19918 or USP 5,990,173) and pharmaceutically acceptable salts thereof (F-12511 is preferred and its chemical name is (S)-2',3',5'-trimethyl-4'-hydroxy-α-dodecylthio-α-phenylacetanilide and F-12511 in the present invention includes its pharmaceutically acceptable salts),
Compounds having the general formula (I) described in Japanese Patent Publication (Kohyo) Hei 10-195037 (EP 790 240 or USP 5,849,732) and pharmaceutically acceptable salts thereof [T-2591 is preferred and its chemical name is 1-(3-tert-butyl-2-hydroxy-5-methoxyphenyl)-3-(2-cyclohexylethyl)-3-(4-dimethylaminophenyl)urea and T-2591 in the present invention includes its pharmaceutically acceptable salts (hydrochloride, etc)],
Compounds having the general formula (I) described in WO 96/26948 and pharmaceutically acceptable salts thereof [FCE-28654 is preferred and its chemical name is 1-(2,6-diisopropylphenyl)-3-[(4R,5R)-4,5-dimethyl-2-(4-phosphonophenyl)-1,3-dioxolan-2-ylmethyl]urea and FCE-28654 in the present invention includes its pharmaceutically acceptable salts (sodium salt, etc)],
Compounds having the general formula (I) described in WO 98/54153 (EP 987 254) and pharmaceutically acceptable salts thereof (K-10085 is preferred and its chemical name is N-[2,4-bis(methylthio)-6-methyl-3-pyridyl]-2-[4-[2-(oxazolo[4,5-b]-pyridin-2-ylthio)ethyl]piperazin-1-yl]acetamide, and K-10085 in the present invention includes its pharmaceutically acceptable salts),
Compounds having the general formula (I) described in WO 92/09572 (EP 559 898 or USP 5,475,130) and pharmaceutically acceptable salts thereof (HL-004 is preferred and its chemical name is N-(2,6-diisopropylphenyl)-2-tetradecyl-thioacetamide),
Compounds having the general formula (I) described in Japanese Patent Publication (Kohyo) Hei 7-82232 (EP 718 281) and pharmaceutically acceptable salts thereof [NTE-122 is preferred and its chemical name is trans-1,4-bis[1-cyclohexyl-3-(4-dimethylaminophenyl)ureidomethyl]cyclohexane, and NTE-122 in the present invention includes its pharmaceutically acceptable salts (hydrochloride, etc)],
Compounds described in Japanese Patent Publication (Kohyo) Hei 10-510512 (WO 96/10559) and pharmaceutically acceptable salts thereof (FR-186054 is preferred and its chemical name is 1-benzyl-1-[3-(pyrazol-3-yl)benzyl]-3-[2,6-bis(methylthio)-4-methylpyridin-3-yl]urea, and FR-186054 in the present invention includes its pharmaceutically acceptable salts),
Compounds having the general formula (I) described in WO 96/09287 (EP 0 782 986 or USP 5,990,150) and pharmaceutically acceptable salts thereof [the preferred compound is N-(1-pentyl-4,6-dimethylindolin-7-yl)-2,2-dimethylpropanamide, and this compound in the present invention includes its pharmaceutically acceptable salts (hydrochloride, etc)], and
Compounds having the general formula (I) described in WO 97/12860 (EP 0 866 059 or USP 6,063,806) and pharmaceutically acceptable salts thereof [the preferred compound is N-(1-octyl-5-carboxymethyl-4,6-dimethylindolin-7-yl)-2,2-dimethylpropanamide and this compound in the present invention includes its pharmaceutically acceptable salts (hydrochloride, sulfate, etc.), and a preferred salt is N-(1-octyl-5-carboxymethyl-4,6-dimethylindolin-7-yl)-2,2-dimethylpropanamide hemisulfate].

Of said ACAT inhibitors, examples of preferred ACAT inhibitors include FR-129169, CI-1011, F-1394, F-12511, T-2591, FCE-28654, K-10085, HL-004, NTE-122, FR-186054, N-(1-pentyl-4,6-dimethylindolin-7-yl)-2,2-dimethylpropanamide and N-(1-octyl-5-carboxymethyl-4,6-dimethylindolin-7-yl)-2,2-dimethylpropanamide.

More preferred compounds are CI-1011, F-12511, N-(1-pentyl-4,6-dimethylindolin-7-yl)-2,2-dimethylpropanamide and N-(1-octyl-5-carboxymethyl-4,6-dimethylindolin-7-yl)-2,2-dimethylpropanamide, and the most preferred compound is N-(1-octyl-5-carboxymethyl-4,6-dimethylindolin-7-yl)-2,2-dimethylpropanamide.

Planar chemical structures of the preferred ACAT inhibitors are shown below.

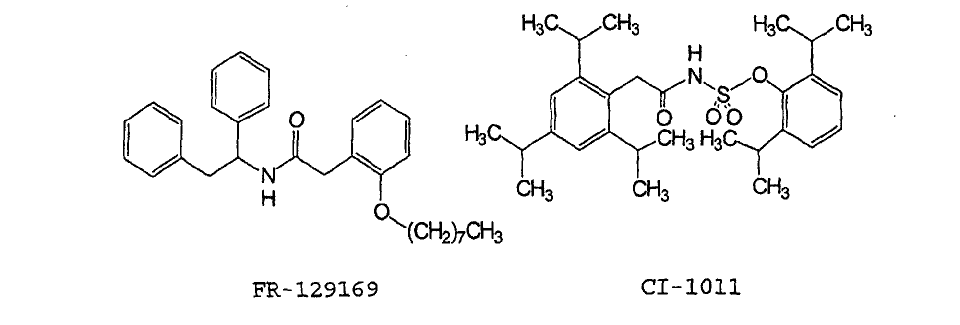

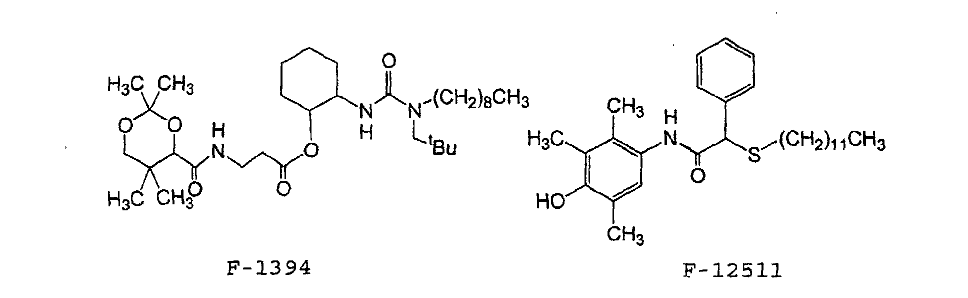

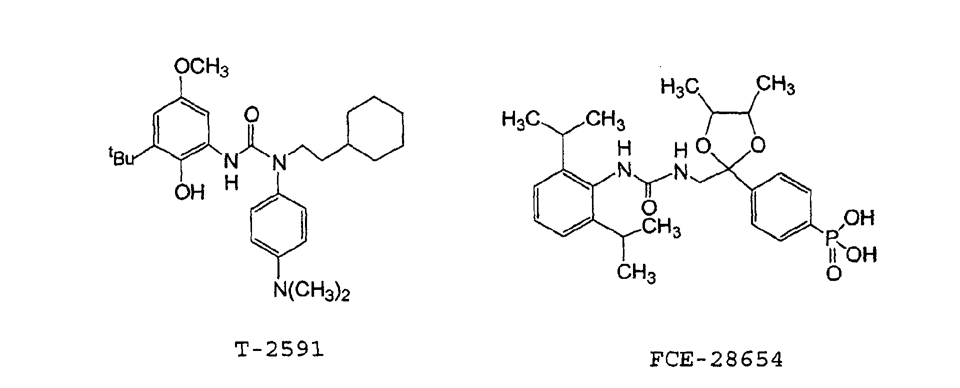

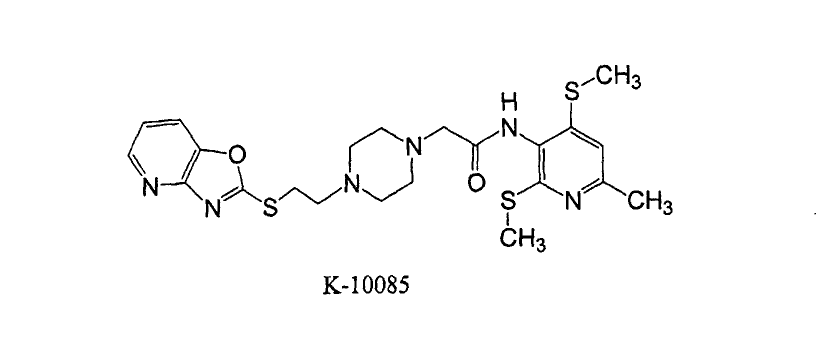

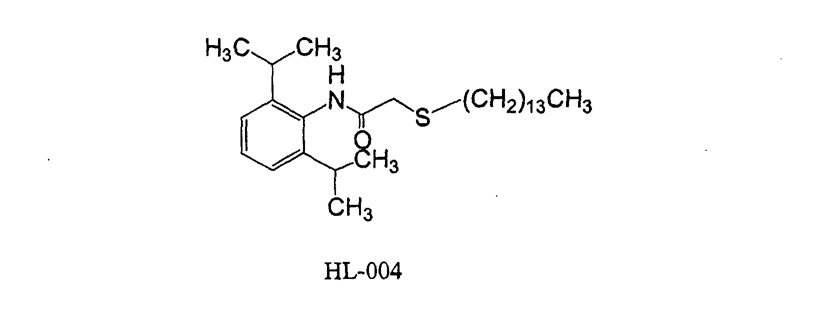

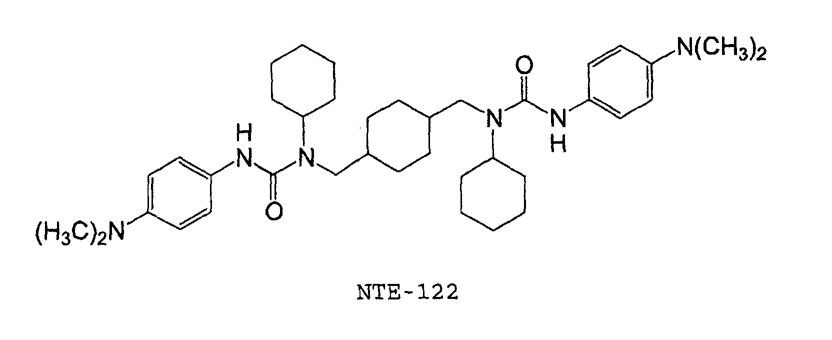

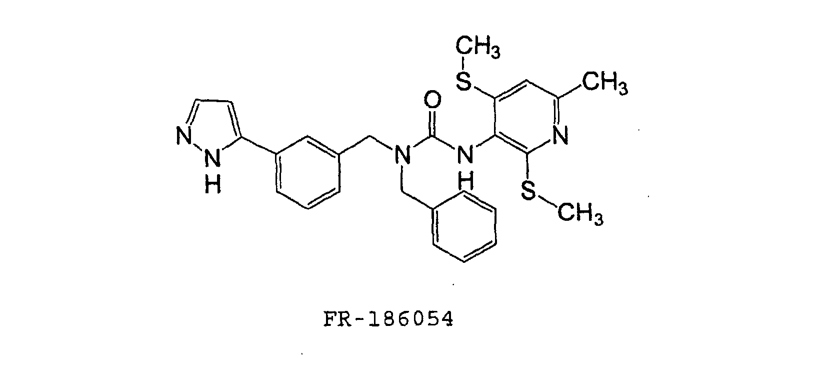

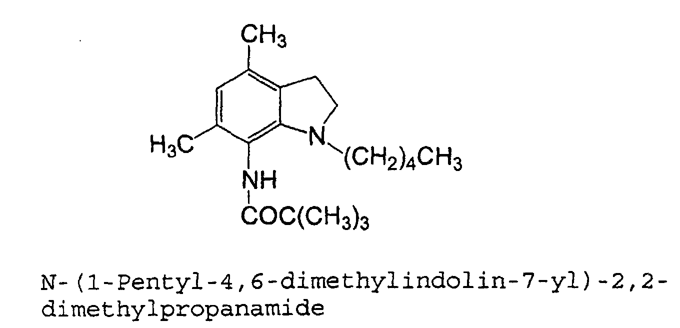

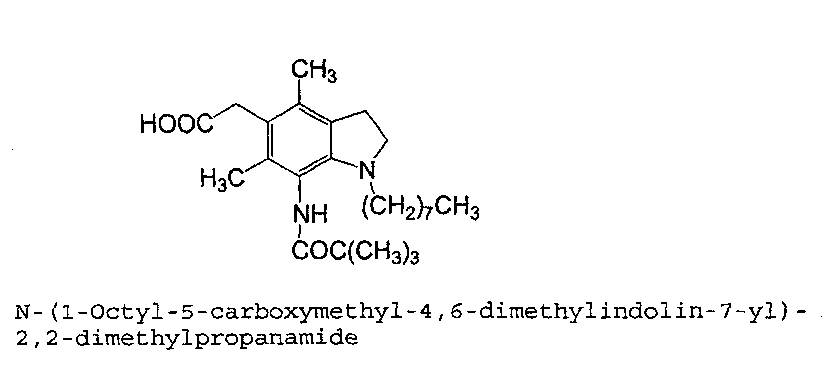

"The insulin resistance reducing agent", which is one of the effective components of the pharmaceutical composition in the present invention, is a therapeutic agent for patients with diabetes mellitus, the mechanism of which is to ameliorate insulin resistance by increasing the sensitivity of insulin to insulin receptors. In the receptors, the agent potentiates tyrosine-kinase activity that activates the signal transduction pathway of the insulin receptors. Thus the agent compensates the functional disorders of insulin receptors. Examples of such insulin resistance reducing agents include oxazole derivatives, oxadiazolidine derivatives, thiazolidine derivatives and phenoxazine derivatives such as
compounds having general formula (I) described in Japanese Patent Publication (Kokai) Sho 61-267580 (EP 193,256 or USP 4,687,777) and pharmaceutically acceptable salts thereof [the preferred compound is pioglitazone and its chemical name is 5-[4-[2-(5-ethyl-pyridin-2-yl)ethoxy]benzyl]-2,4-thiazolidinedione and pioglitazone in the present invention includes its pharmaceutically acceptable salts (hydrochloride, etc.)],
compounds having general formula (I) described in Japanese Patent Publication (Kohyo) Hei 9-512249 (WO 95/21608 or USP 5,002,953) and pharmaceutically acceptable salts thereof [the preferred compound is rosiglitazone and its chemical name is 5-[4-[2-(N-methyl-N-2-pyridylamino)ethoxy]benzyl]-2,4-thiazolidinedione, and rosiglitazone in the present invention includes its pharmaceutically acceptable salts (maleate, etc)],
compounds having general formula (I) described in WO 00/8002 and pharmaceutically acceptable salts thereof [the preferred compound is GI-262570 and its chemical name is 2(S)-(2-benzoylphenylamino)-3-[4-[2-(5-methyl-2-phenyloxazol-4-yl)ethoxy]phenyl]propionic acid, and GI-262570 in the present invention includes its pharmaceutically acceptable salts (hydrochloride, etc)],
compounds having general formula (I) described in WO 95/18125 (EP 684,242 or USP 5,728,720) and pharmaceutically acceptable salts thereof [the preferred compound is JTT-501 and its chemical name is 4-[4-[2-(5-methyl-2-phenyl-oxazol-4-yl)ethoxy]benzyl-3,5-isoxazolidinedione, and JTT-501 in the present invention includes its pharmaceutically acceptable salts (hydrochloride, etc)],
compounds having general formula (I) described in WO 99/62872 (EP 1,084,103 or USP 6,258,850) and pharmaceutically acceptable salts thereof [the preferred compound is AZ-242 and its chemical name is 2-ethoxy-3-[4-[2-[4-(methylsulfonyloxy)phenyl]ethoxy]phenyl]propanoic acid, and AZ-242 in the present invention includes its pharmaceutically acceptable salts (sodium salt, etc.),
compounds having general formula (I) described in Japanese Patent Publication (Kokai) Hei 6-247945 (EP 604,983 or USP 5,594,016) and pharmaceutically acceptable salts thereof (the preferred compound is MCC-555 and its chemical name is 5-[6-(2-fluorobenzyloxy)naphthalen-2-ylmethyl]thiazolidine-2,4-dione, and MCC-555 in the present invention includes its pharmaceutically acceptable salts),
compounds having general formula (I) described in WO 94/25448 (EP 696,585 or USP 5,643,931) and pharmaceutically acceptable salts thereof (the preferred compound is YM-440 and its chemical name is (Z)-1,4-bis[4-[(3,5-dioxo-1,2,4-oxadiazolidin-2-yl)methyl]phenoxy]-2-butene, and YM-440 in the present invention includes its pharmaceutically acceptable salts],
compounds having general formula (I) described in Japanese Patent Publication (Kokai) Hei 10-87641 (USP 5,948,803) and pharmaceutically acceptable salts thereof (the preferred compound is KRP-297 and its chemical name is 5-(2,4-dioxothiazolidin-5-ylmethyl)-2-methoxy-N-(4-trifluoromethylbenzyl)benzamide, and KRP-297 in the present invention includes its pharmaceutically acceptable salts),
compounds having general formula (I) described in Japanese Patent Publication (Kokai) Sho 64-56675 (EP 283035 or USP 4,897,393) and pharmaceutically acceptable salts thereof [the preferred compound is T-174 and its chemical name is 5-[2-(naphthalene-2-ylmethyl)benzooxazol-5-ylmethyl]-2,4-thiazolidinedione, and T-174 in the present invention includes its pharmaceutically acceptable salts (hydrochloride, etc)],
compounds having general formula (I) described in Japanese Patent Publication (Kokai) Hei 9-100280 (EP 787725 or USP 5,693,651) and pharmaceutically acceptable salts thereof [the preferred compound is NC-2100 and its chemical name is 5-(7-benzyloxy-3-quinoylmethyl)-2,4-thiazolidinedione, and NC-2100 in the present invention includes its pharmaceutically acceptable salts (hydrochloride, etc)],
compounds having general formula (I) described in WO 99/19313 (USP 6,054,453) and pharmaceutically acceptable salts thereof [the preferred compound is NN-622 and its chemical name is (S)-3-[4-[2-(phenoxazin-10-yl)ethoxy]phenyl]-2-ethoxypropanoic acid, and NN-622 in the present invention includes its pharmaceutically acceptable salts (sodium salt, etc)],
compounds having general formula (I) described in WO 01/21602 and pharmaceutically acceptable salts thereof [the preferred compound is BMS-298585 and its chemical name is N-(4-methoxyphenoxycarbonyl)-N-[4-[2-(5-methyl-2-phenyl-4-oxazolyl)ethoxy]benzyl]glycine, and BMS-298585 in the present invention includes its pharmaceutically acceptable salts (hydrochloride, etc)], and
compounds having general formula (I) described in Japanese Patent Publication (Kokai) Hei 9-295970 (USP 5,886,014) and pharmaceutically acceptable salts thereof [the preferred compound is 5-[4-(6-methoxy-1-methylbenzimidazol-2-ylmethoxy)-benzyl]thiazolidine-2,4-dione, and this compound includes its pharmaceutically acceptable salts (hydrochloride, etc)]. Preferred compounds are pioglitazone, rosiglitazone, GI-262570, JTT-501, AZ-242, MCC-555, YM-440, KRP-297, T-174, NC-2100, NN-622, BMS-298585, and 5-[4-(6-methoxy-1-methylbenzimidazol-2-ylmethoxy)benzyl]thiazolidine-2,4-dione. More preferred compounds are pioglitazone, rosiglitazone and 5-[4-(6-methoxy-1-methylbenzimidazol-2-ylmethoxy)benzyl]-thiazolidine-2,4-dione, and the most preferred compound is 5-[4-(6-methoxy-1-methylbenzimidazol-2-ylmethoxy)benzyl]thiazolidine-2,4-dione.

Planar chemical structures of the preferred insulin resistance reducing agents are shown below.

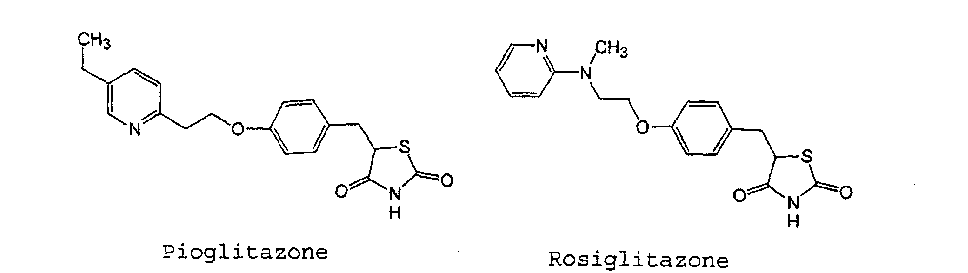

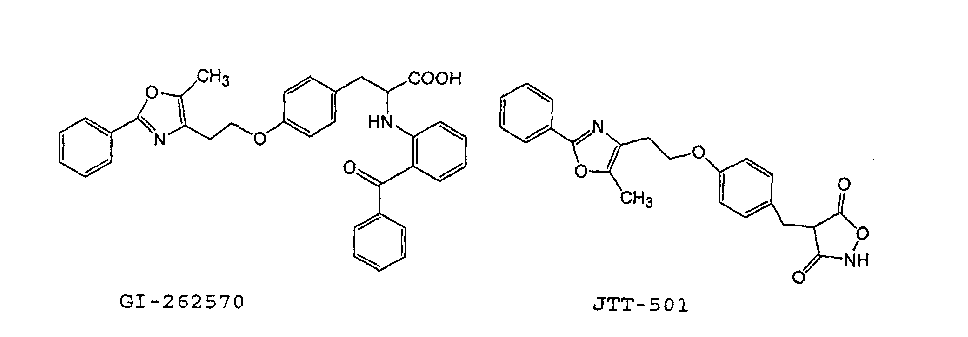

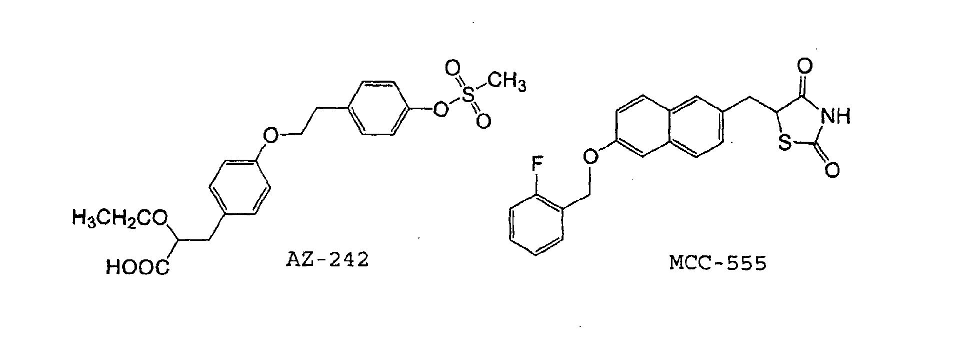

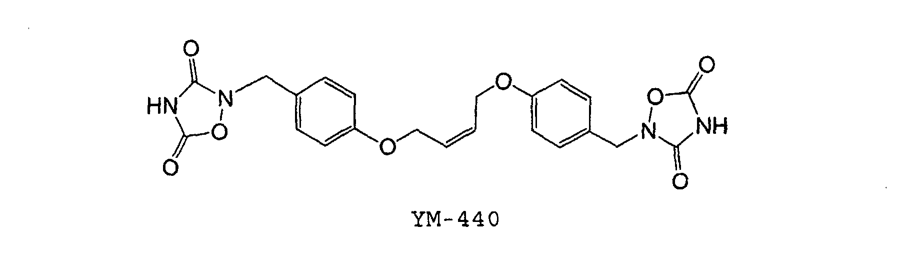

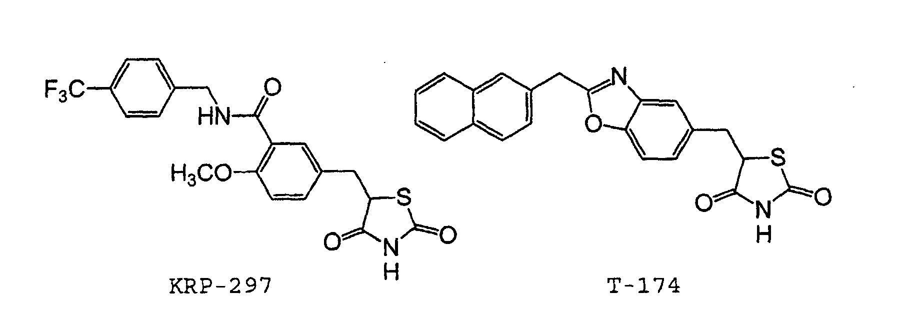

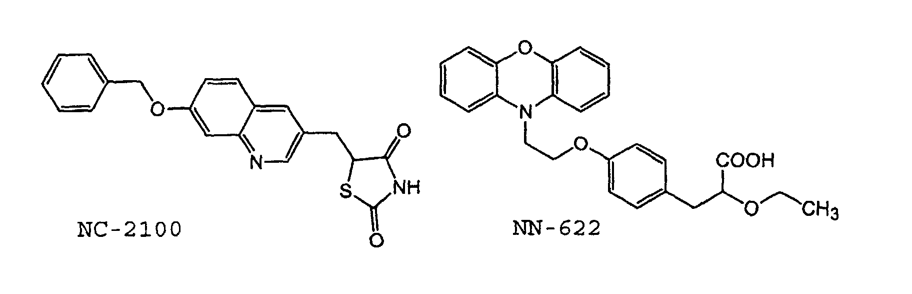

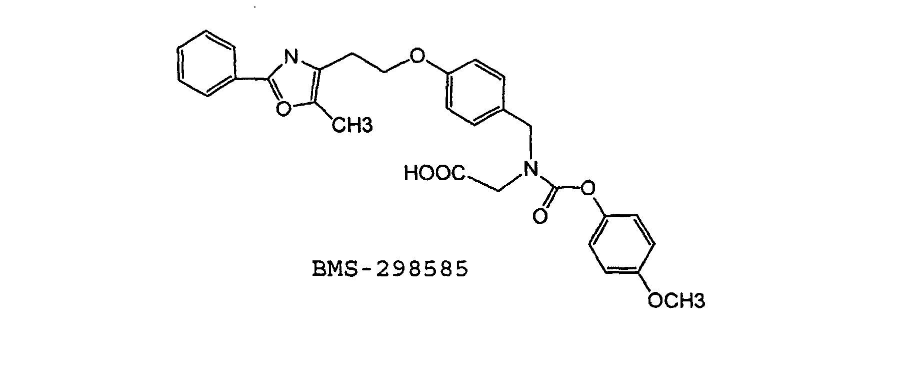

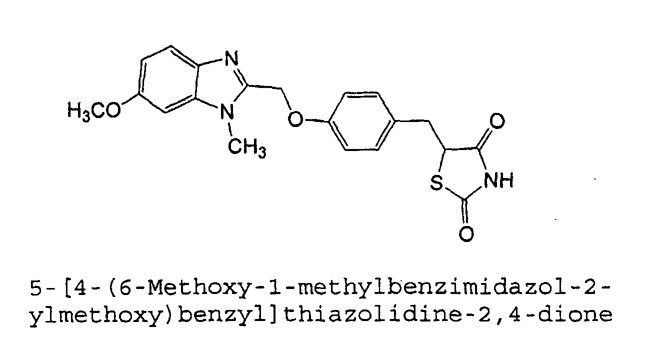

The "pharmaceutically acceptable salt" described above can be prepared in a conventional manner by reaction with an acid if the ACAT inhibitors and/or insulin resistance reducing agents contained as the active components have a basic group such as an amino group or by reaction with a base if these active components have an acidic group such as a carboxyl group.

Examples of the salts depending on the basic group in said effective components include, but are not limited to, the salts of halogenated hydroacids such as hydrofluoride, hydrochloride, hydrobromide and hydroiodide; inorganic acids such as nitrate, perchlorate, sulfate and phosphate; lower alkanesulfonic acids such as methanesulfonate, trifluoromethanesulfonate and ethanesulfonate; arylsulfonic acids such as benzenesulfonate and p-toluenesulfonate; organic acids such as acetate, malate, fumarate, succinate, citrate, ascorbate, tartrate, oxalate and maleate; and amino acid derivatives such as glycine salt, lysine salt, arginine salt, omithine salt, glutamate and aspartate. Of these salts, preferred salts are hydrochloride, sulfate, acetate, fumarate, succinate and maleate.

On the other hand, examples of the salts depending on the acidic group in said effective components include the salts of alkali metals such as sodium salt, potassium salt and lithium salt; alkaline earth metals such as calcium salt and magnesium salt; metals salts such as aluminum salt and iron salt; inorganic amines salts such as ammonium salt; organic amines salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N-dibenzylethylenediamine salt, chloroprocaine salt, procaine salt, diethanolamine salt, N-benzylphenethylamine salt, piperazine salt, tetramethylammonium salt and tris(hydroxymethyl)aminomethane salt; and amino acid derivative salts such as glycine salt, lysine salt, arginine salt, ornithine salt, glutamate and aspartate. Of these salts, more preferred salts are sodium salt, potassium salt, calcium salt, magnesium salt and aluminum salt.

The "solvate" described above is an adduct formed when the solvent, for example water and/or alcohol, that was used in the manufacturing or purification process is absorbed by the ACAT inhibitor and/or insulin resistance reducing agent, and is not particularly limited provided that it has no adverse effect on the ACAT inhibitor and/or insulin resistance reducing agent. When ACAT inhibitors and insulin resistance reducing agents are allowed to stand in the atmosphere, these active components may absorb water or be attached by adsorbed water to form their hydrates, which are also included in said "solvate". Of these solvates, the preferred solvate is a hydrate.

When asymmetric carbon atoms are present in the molecules of the ACAT inhibitors and insulin resistance reducing agents that are the active components of the pharmaceutical composition of the present invention, various kinds of isomers exist. The compounds contained in the present invention, including all of these isomers and their mixtures, are represented by a single general formula for each active component. Accordingly, the present invention also encompasses all of these isomers and isomer mixtures with all the possible component ratios.

The pharmaceutical composition of the present invention comprising an ACAT inhibitor and an insulin resistance reducing agent is a composition that is characterized by the administration of these two components at the same time or independently at a particular time interval.

According to the present invention, both the ACAT inhibitor and the insulin resistance reducing agent exert more potent therapeutic efficacies by co-administration rather than by administration of either agent alone. Furthermore, each component of the pharmaceutical composition is not necessary to be present in the body at the same time for exerting such potent therapeutic efficacies. In other words, augmentation of efficacy can be observed even if both the ACAT inhibitor and insulin resistance reducing agent do not have sufficiently high plasma levels concurrently. However, since it is clinically convenient to administer the ACAT inhibitor and the insulin resistance reducing agent at the same time, both of these two agents can be administered as a single pharmaceutical composition. However, in cases where it is unfavorable to mix these two components physically from the formulating technical point of view, each component can be administered separately at the same time. In addition, since the ACAT inhibitor and the insulin resistance reducing agent exert excellent efficacies even when not administered at the same time, these two components can be administered at particular intervals; furthermore, either agent can be administered prior to the other.

Thus the co-administration described in the present invention is not limited as far as the dosage forms of these agents are suitable for administration roughly at the same time, but it is desirable to administer these two agents as a single pharmaceutical composition.

On the other hand, the separate administration of these two agents within a particular time interval, which is described in the present invention, is not limited as far as the dosage forms of these agents that are suitable for independent administration at different times. For example, the ACAT inhibitor can be administered first then the insulin resistance reducing agent later at a particular time interval, or the insulin resistance reducing agent is administered first then the ACAT inhibitor may be administered later at a particular time interval. The maximum time interval between administrations of the ACAT inhibitor and the insulin resistance reducing agent that is acceptable for achieving excellent therapeutic efficacy by these two agents can be confirmed by clinical studies and/or animal experiments.

The ACAT inhibitor, which is one of active components of the pharmaceutical composition of the present invention, can be easily manufactured by methods described, for example, in WO 92/09561, Japanese Patent Publication (Kohyo) Hei 8-510256 (WO 94/26702, USP 5,491,172), EP 421 441 (USP 5,120,738), Japanese Patent Publication (Kohyo) 2000-50771 (WO 97/19918, USP 5,990,173), Japanese Patent Publication (Kokai) Hei 10―195037 (EP 790 240, USP 5,849,732), WO 96/26948, WO 98/54153 (EP 987 254), WO 92/09572 (EP 559 898, USP 5,475,130), Japanese Patent Publication (Kokai) Hei 7-82232 (EP 718 281), Japanese Patent Publication (Kohyo) Hei 10-510512 (WO 96/10559), WO 96/09287 (EP 0 782 986, USP 5,990,150), and WO 97/12860 (EP 0 866 059, USP 6,063,806).

On the other hand, the insulin resistance reducing agent, which is an active component of the pharmaceutical composition of the present invention, can be also easily manufactured by methods described, for example, in Japanese Patent Publication (Kokai) Sho 61-267580 (EP 193 256, USP 4,687,777), Japanese Patent Publication (Kohyo) Hei 9-512249 (WO 95/21608, USP 5,002,953), WO 00/8002, WO 95/18125 (EP 684 242, USP 5,728,720), WO 99/62872 (EP 1 084 103, USP 6,258,850), Japanese Patent Publication (Kokai) Hei 6-247945 (EP 604 983, USP 5,594,016), WO 94/25448 (EP 696 585, USP 5,643,931), Japanese Patent Publication (Kokai) Hei 10-87641 (USP 5,948,803), Japanese Patent Publication (Kokai) Sho 64-56675 (EP 283 035, USP 4,897,393), Japanese Patent Publication (Kokai) Hei 9-100280 (EP 787 725, USP 5,693,651), WO 99/19313 (USP 6,054,453), WO 01/21602, and Japanese Patent Publication (Kokai) Hei 9-295970 (USP 5,886,014).

### [Best mode for carrying out the invention]

The present invention is illustrated in more detail by the following test examples and formulation examples. However, the present invention is not limited to these examples.

### Test Example 1

### Determination of Areas of Pathological Changes Appearing in the Aorta

Male apolipoprotein E-deficient mice of 12 weeks of age were divided into 4 groups (10 mice per a group) as follows: control group, group treated with 5-[4-(6-methoxy-1-methylbenzimidazol-2-yl-methoxy)benzyl]thiazolidine-2,4-dione hydrochloride (mixed with diet at a concentration of 0.0001 w/w%, hereinafter referred to as Compound B), group treated with N-(1-octyl-5-carboxymethyl-4,6-dimethylindolin-7-yl)-2,2-dimethylpropanamide hemisulfate (mixed with diet at a concentration of 0.03 w/w%, hereinafter referred to as Compound A), and co-administration group of Compound B (mixed with diet at a concentration of 0.0001 w/w%) and Compound A (mixed with diet at a concentration of 0.03 w/w%). All mice were treated with compounds mixed with diet except the control group for 12 successive weeks. The mice were autopsied in the 12th week and the area of atherosclerotic lesions in the aortic valve and the ratio of the area occupied by macrophages in the same area (positively stained area with anti-MOMA-2) were determined.

### (1) Areas of Atherosclerotic Lesions in the Aorta

The aortic valve was stained with elastic-Masson and the area of atherosclerotic lesions in the aortic valve determined. Less-stained areas indicate greater inhibitory effects of the agents on the atherosclerotic lesions as well as suppressive action of the agents against progressive development of atherosclerotic lesions in the aorta.

### (2) Rate of Area Occupied by Macrophages

Macrophages in the aortic valve were detected by staining with anti-MOMA-2. Areas occupied by macrophages in the aortic valve were determined and the ratio of the area occupied by macrophages (positively stained areas with anti-MOMA-2) to the area of atherosclerotic lesions in the aortic valve was calculated.

Macrophages are a factor to make plaques unstable. Thus decreases in macrophage accumulation increase plaque stability and are related to amelioration of the atherosclerotic lesions. This indicates that compounds that reduced areas occupied by macrophages exhibit suppressive effects on progressive development of atherosclerosis.

The results obtained from experiments (1) and (2) are summarized in Table 1. The actual measurement value in Table 1 indicates average ± standard error.

**[Table 1]**

| Group | Area of atherosclerotic lesions (µm²) | Rate of area occupied with macrophage (%) |
|---|---|---|
| Control | 127246 ± 20950 | 20.2 ± 3.6 |
| Group treated with Compound B | 80616 ± 11611 | 23.3 ± 4.1 |
| Group treated with Compound A | 65496* ± 15344 | 13.7 ± 2.2 |
| Group treated with Compounds A and B | 70467 ± 12132 | 2.8 ± 0.6** |

| | | |
|---|---|---|
| *) Value in the group treated with Compound A was significantly (p≤0.05) different from that in the control group by Dunnett's multiple comparison test. | | |
| **) Value in the group treated with Compounds A and B was significantly (p≤0.01) different from that in the control group by Dunnett's multiple comparison test. | | |

### [Formulation Example]

| Tablets | |
|---|---|
| Compound B | 50.0 mg |
| Compound A | 10.0 mg |
| Lactose | 113.0 mg |
| Corn Starch | 25.0 mg |
| Magnesium stearate | 2.0 mg |
| | 200 mg |

The powders described above are mixed well and tableted with a tableting machine to prepare a tablet comprising 200 mg of the composition. Compound A in the above prescription indicates N-(1-octyl-5-carboxymethyl-4,6-dimethylindolin-7-yl)-2,2-dimethylpropanamide hemisulfate, while Compound B indicates 5-[4-(6-methoxy-1-methylbenzimidazol-2-ylmethoxy)benzyl]thiazolidine-2,4-dione hydrochloride.

### [Possibility of Industrial Utilization]

The pharmaceutical composition of the present invention comprising an ACAT inhibitor and an insulin resistance reducing agent exhibits excellent suppressive effects on the progressive development of atherosclerosis and on the appearance of xanthomatosis in the arthrosis of four limbs. In addition, the pharmaceutical composition has low toxicity. Thus the pharmaceutical composition is useful as a prophylactic or therapeutic agent (particularly as a therapeutic agent) against atherosclerosis or diseases caused by atherosclerosis such as ischemic heart disease, ischemic cerebral disease and peripheral circulatory disorders in warm-blooded animals (particularly in humans).

The ACAT inhibitor and the insulin resistance reducing agent, which are active components of the present invention, can be prepared as independently administrable dosage forms or as a singly administrable dosage form by mixing these two agents physically as described above.

In cases where the pharmaceutical composition of the present invention is used as a prophylactic or therapeutic agent against the disorders described above, the ACAT inhibitor and insulin resistance reducing agent which are the effective components of the present invention may be administered in dosage forms for oral administration such as tablets, capsules, granules, powders, or syrups, or dosage forms for non-oral administration such as injections, or dosage forms for external application such as suppositories by optionally mixing with pharmacologically acceptable excipients and diluents, etc.

These formulations are prepared by conventionally known methods using additives such as excipients (for instance, organic excipients including sugar derivatives such as lactose, sucrose, glucose, mannitol and sorbitol; starch derivatives such as corn starch, potato starch, α-starch and dextrin; cellulose derivatives such as crystalline cellulose; gum Arabic; dextran; pullulan; and inorganic excipients including silicate derivatives such as light silicic anhydride, synthetic aluminum silicate, calcium silicate and magnesium aluminometasilicate; phosphates such as calcium hydrogenphosphate; carbonates such as calcium carbonate; and sulfates such as calcium sulfate can be listed), lubricants (for instance, stearic acid, metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; sodium salts of fatty acids; lauryl sulphates such as sodium lauryl sulfate and magnesium lauryl sulfate; silicates such as silicic anhydride and silicic hydrate; and starch derivatives described above can be listed), binders (for instance, hydroxypropylcellulose, hydroxypropylmethylcellulose, polyvinylpyrrolidone, Macrogol and similar excipients described above can be listed), disintegrants (for instance, cellulose derivatives such as low-substituted hydroxypropylcellulose, carboxymethylcellulose, calcium carboxymethylcellulose and internally crosslinked-sodium carboxymethylcellulose; chemically modified starch/cellulose derivatives such as carboxymethylstarch, sodium carboxymethylstarch, crosslinked polyvinylpyrrolidone can be listed), stabilizers (for instance, para-oxy benzoates such as methylparaben and propylparaben; alcohols such as chlorobutanol, benzylalcohol and phenylethylalcohol; benzalkonium chloride; phenols such as phenol and cresol; thimerosal; dehydroacetic acid; and sorbic acid can be listed), flavors (for instance, conventionally employed sweeteners, acidifiers and flavors can be listed), and diluents, etc.

The dosage and the dosage frequency of the ACAT inhibitor and the insulin resistance reducing agent in the present invention varies depending on the activity of each agent, the symptoms, age, body weight, etc. of the patient.

The dosage varies depending on the symptoms, age, etc. of the patient. In the case of oral administration, it is desirable to administer 0.1 mg (preferably 0.5 mg) as a lower limit and 1000 mg (preferably 500 mg) as an upper limit of each agent per one time, while in the case of non-oral administration, it is desirable to administer 0.01 mg (preferably 0.05 mg) as a lower limit and 100 mg (preferably 50 mg) as an upper limit of each agent per one time for an adult and at a frequency of one to six times a day, at the same time or separately at a particular time interval depending on the symptoms.

Furthermore, the ratio of the dosage of ACAT inhibitor to that of insulin resistance reducing agent may vary considerably. For instance, the weight ratio of the dosage of the ACAT inhibitor to that of the insulin resistance reducing agent could be set in a range extending from 1:500 to 500:1.

## Claims

1. A pharmaceutical composition comprising an ACAT inhibitor and an insulin resistance reducing agent for the prevention or therapy of atherosclerosis or diseases caused by atherosclerosis, said composition being formulated to provide administration of these two agents at the same time or independently at a particular time interval.

2. The pharmaceutical composition according to claim 1, wherein the ACAT inhibitor is FR-129169, CI-1011, F-1394, F-12511, T-2591, FCE-28654, K-10085, HL-004, NTE-122, FR-186054, N-(1-pentyl-4,6-dimethylindolin-7-yl)-2,2-dimethylpropanamide or N-(1-octyl-5-carboxymethyl-4,6-dimethylindolin-7-yl)-2,2-dimethylpropanamide, or a pharmaceutically acceptable salt thereof.

3. The pharmaceutical composition according to claim 1, wherein the ACAT inhibitor is CI-1011, F-12511, N-(1-pentyl-4,6-dimethylindolin-7-yl)-2,2-dimethylpropanamide or N-(1-octyl-5-carboxymethyl-4,6-dimethylindolin-7-yl)-2,2-dimethylpropanamide, or a pharmaceutically acceptable salt thereof.

4. The pharmaceutical composition according to claim 1, wherein the ACAT inhibitor is N-(1-octyl-5-carboxymethyl-4,6-dimethylindolin-7-yl)-2,2-dimethylpropanamide or a pharmaceutically acceptable salt thereof.

5. The pharmaceutical composition according to any one of claims 1 to 4, wherein the insulin resistance reducing agent is pioglitazone, rosiglitazone, GI-262570, JTT-501, AZ-242, MCC-555, YM-440, KRP-297, T-174, NC-2100, NN-622, BMS-298585 or 5-[4-(6-methoxy-1-methylbenzimidazol-2-ylmethoxy)benzyl]thiazolidine-2,4-dione, or a pharmaceutically acceptable salt thereof.

6. The pharmaceutical composition according to any one of claims 1 to 4, wherein the insulin resistance reducing agent is pioglitazone, rosiglitazone or 5-[4-(6-methoxy-1-methylbenzimidazol-2-ylmethoxy)benzyl]thiazolidine-2,4-dione, or a pharmaceutically acceptable salt thereof.

7. The pharmaceutical composition according to any one of claims 1 to 4, wherein the insulin resistance reducing agent is 5-[4-(6-methoxy-1-methylbenzimidazol-2-ylmethoxy)benzyl]thiazolidine-2,4-dione or a pharmaceutically acceptable salt thereof.

8. The pharmaceutical composition according to any one of claims 1 to 7 for the prevention or therapy of atherosclerosis.

9. The pharmaceutical composition according to any one of claims 1 to 7 for the prevention or therapy of diseases caused by atherosclerosis.

10. The pharmaceutical composition according to claim 9, wherein the diseases caused by atherosclerosis are ischemic heart diseases.

11. The pharmaceutical composition according to claim 9, wherein the diseases caused by atherosclerosis are ischemic brain diseases.

12. The pharmaceutical composition according to claim 9, wherein the diseases caused by atherosclerosis are peripheral circulatory disorders.
